Europäisches Patentamt

European Patent Office

Office européen des brevets

(11) Numéro de publication : **0 190 990**
**B1**

(12) **FASCICULE DE BREVET EUROPÉEN**

(45) Date de publication du fascicule du brevet :
09.08.89

(21) Numéro de dépôt : 86810032.2

(22) Date de dépôt : 21.01.86

(51) Int. Cl.⁴ : **A 61 B 17/60**

(54) Elément d'arceau et fixateur externe pour ostéosynthèse et ostéoplastie.

(30) Priorité : 24.01.85 CH 309/85

(43) Date de publication de la demande :
13.08.86 Bulletin 86/33

(45) Mention de la délivrance du brevet :
09.08.89 Bulletin 89/32

(84) Etats contractants désignés :
**AT BE DE FR GB IT LU NL SE**

(56) Documents cités :
**EP--A-- 0 029 298**
**GB--A-- 2 031 731**
**GB--A-- 2 114 891**

(73) Titulaire : **JAQUET ORTHOPEDIE S.A.**
**5, chemin des Aulx**
**CH-1228 Plan-les-Ouates (CH)**

(72) Inventeur : **Monticelli, Georgio, Prof.**
**Université de Rome**
**I-00147 Rome (IT)**
Inventeur : **Spinelli, Renato, Prof.**
**Via del Casale de Merode 7**
**I-00147 Rome (IT)**
Inventeur : **Wagenknecht, Marcel**
**chemin des Milans 12**
**CH-1219 Le Lignon (CH)**

(74) Mandataire : **Dietlin, Henri et al**
**Dietlin & Cie S.A. Rue des Epinettes 19**
**CH-1227 Genève (CH)**

Jouve, 18, rue St-Denis, 75001 Paris, France

## Description

L'invention a pour objet un élément d'arceau destiné à être utilisé en ostéosynthèse et en ostéoplastie. Elle vise également un fixateur externe dans lequel l'élément d'arceau est incorporé lors de l'utilisation.

La fixation externe en chirurgie est une ancienne méthode, qui date d'un siècle, et son application a été limitée longtemps aux fractures compliquées en traumatologie et par la suite en orthopédie, c'est-à-dire dans les traitements secondaires des fractures, infections, retards de consolidation, pseudarthoses, mauvaises réductions, etc.

La fixation externe s'adresse plus spécialement aux os longs, tels que le fémur, le tibia, l'humérus, le radius, le cubitus et particulièrement à la jambe.

La titulaire a développé un nouveau système de fixateurs externes avec extension de leur application au bassin, à la clavicule, aux articulations telles que genou, coude, épaule et au halo pour traitement des vertèbres cervicales et lombaires, etc.

Les fixateurs externes pour ostéosynthèse permettent deux sortes de fixation de l'os :
- la fixation transfixiante où les fiches traversent le membre de part en part,
- la fixation non transfixiante où les fiches sont introduites dans l'os sans le traverser.

La fixation transfixiante, qui est la plus utilisée sur la jambe, est plus rigide que la fixation non transfixiante.

De chaque côté de la jambe, on monte deux tiges ou deux cadres, qui viennent se fixer sur deux groupes de fiches, placés de part et d'autre de la fracture. Les deux cadres ou les deux tiges sont reliés par une armature aussi stable que possible, qui peut comprendre des tiges ou des barres à coulisse dont la longueur est susceptible d'être augmentée ou diminuée, opérant ainsi une extension ou une compression, respectivement.

On connaît déjà, par le brevet européen EP-A-0.029.298, des arceaux présentant une section en I destinée à créer des bordures de renfort de part et d'autre de la partie centrale plane, laquelle comporte des ouvertures destinées à la fixation des pièces intermédiaires de montage. L'emplacement pré-déterminé de ces ouvertures limite les possibilités d'assemblage.

Dans le brevet GB-A-2.031.731, on propose un fixateur comportant des arceaux présentant des ouvertures pour le passage d'éléments de serrage des composants, dont le positionnement est de ce fait limité.

Le brevet GB-2.114.891 de la déposante proposait dans la variante des figures 3 et 4 un arceau de section triangulaire pour le montage de pièces intermédiaires qui permet :

a) de disposer à l'avance sur l'arceau les différentes pièces intermédiaires dont le montage est prévu, sur la bordure intérieure ou extérieure de l'arceau ;

b) de positionner celles-ci grossièrement au début de la mise en place du fixateur externe, pour maintenir les composants en place tout en autorisant encore un réglage fin de leur position ;

c) de les bloquer enfin lorsque la position optimale est atteinte.

La présente invention a pour objet d'augmenter les possibilités d'application du système selon le brevet précité. En particulier, il s'agit de créer des possibilités multiples et étendues de montage des tiges, des fiches et d'autres pièces et organes de liaison sur l'élément d'arceau destiné à entourer l'os en partie ou en totalité.

Ce but est rempli par le fixateur externe pour ostéosynthèse et ostéoplastie selon l'invention qui comprend au moins deux groupes d'au moins une fiche ou un fil maintenant chacun un fragment osseux, des tiges d'assemblages, au moins un élément d'arceau toroïdal ainsi que des organes de fixation des fiches ou fils et des tiges d'assemblage. Il est caractérisé par le fait que la section dudit élément d'arceau comprend deux renforts latéraux de section polygonale réunis par un profil de liaison et d'écartement, lesdits renforts latéraux de section polygonale étant destinés à recevoir lesdits moyens de fixation, qui sont adaptés à être montés, déplacés librement, puis bloqués en toute position sur l'un et/ou l'autre des renforts constituant l'élément d'arceau.

Ce type d'arceau a pour avantage une mise en place dans un temps plus réduit que les fixateurs connus précédemment, ce qui a deux conséquences importantes :
- la diminution du temps d'anesthésie du patient,
- la réduction des frais d'opération, qu'il s'agisse du personnel médical ou des installations en salle d'opération.

Des exécutions particulières ou préférées font l'objet des revendications dépendantes.

Les pièces-supports et/ou les pièces d'assemblage comprennent normalement des mâchoires présentant des découpures internes agencées pour épouser au moins une partie du pourtour de l'arceau, de manière à pouvoir être pressées et bloquées sur l'arceau sans possibilité de rotation autour de celui-ci, et chaque pièce d'assemblage et/ou chaque pièce-support peut comprend un alésage dans lequel est introduite la tige, la fiche ou le fil.

Cependant, puisque l'élément d'arceau comprend deux sections polygonales, en règle générale dans un plan horizontal du tore formant l'arceau, un polygone se trouvant à l'extérieur et l'autre à l'intérieur du tore, les pièces d'assemblage peuvent être fixées à l'intérieur et à l'extérieur de l'arceau, même sur le même rayon.

L'alésage de chaque pièce d'assemblage ou de support peut être muni d'une douille ou canon agencé pour adapter le diamètre des fils et fiches à l'alésage de la pièce.

L'alésage peut être pratiqué de manière que son axe soit radial par rapport à l'arceau lorsque la pièce y est fixée, ou de manière que son axe soit perpendiculaire au plan de l'arceau lorsque la pièce y est fixée.

La pièce d'assemblage peut présenter une partie pivotant autour d'une vis de serrage relativement à l'ensemble de la pièce, la partie pivotante comprenant l'alésage et la partie pivotante peut être réalisée à partir d'une bride qui comporte un alésage et une fente.

Selon un mode d'exécution préféré, des fiches supplémentaires destinées à maintenir des fragments d'os supplémentaires sont fixées sur l'arceau à l'aide de dispositifs adéquats agencés pour positionner le fragment supplémentaire latéralement par rapport à l'arceau.

Les deux parties polygonales de la section de l'arceau peuvent être identiques ou différentes. Elles peuvent être situées dans le même plan horizontal, ce qui est préféré, ou décalées verticalement l'une par rapport à l'autre. Les deux parties polygonales sont normalement identiques pour qu'on puisse utiliser les mêmes pièces d'assemblage. De préférence, la section complète est formée de deux parties triangulaires et un profil de liaison et d'écartement rectangulaire, les triangles étant sensiblement isocèles et dont les sommets sont dirigés l'un vers l'autre et confondus dans le profil de liaison.

L'élément d'arceau peut former une partie d'un cercle, par exemple un quart, un demi-cercle ou les trois quarts, ou bien un cercle complet. Une exécution est prévue dans laquelle l'élément d'arceau forme les deux tiers jusqu'à environ les trois quarts d'un cercle, un cercle complet pouvant être constitué par une autre pièce présentant le même profil que la première et pouvant également servir comme élément d'arceau.

Le fixateur selon l'invention présentent les avantages nommés dans le brevet GB-2.114.891. De plus, il se prête à un montage plus dense des pièces d'assemblage et des supports des fiches et tiges. On a également constaté une plus grande rigidité de l'arceau, ce qui est évidemment déterminant pour le succès des opérations de compression et d'extension de l'os.

Le dessin représente, à titre d'exemples, des formes de réalisation pratique. Dans le dessin, les figures représentent :

- la fig. 1 : une vue en perspective de deux éléments d'arceau,
- la fig. 2 : une coupe verticale du tore formant l'arceau, montrant la section,
- la fig. 3 : une vue en perspective du fixateur, monté autour d'un os,
- la fig. 4 : l'application d'un élément d'arceau formant un cercle partiel, et
- la fig. 5 : l'utilisation de trois éléments d'arceau formant des cercles complets.

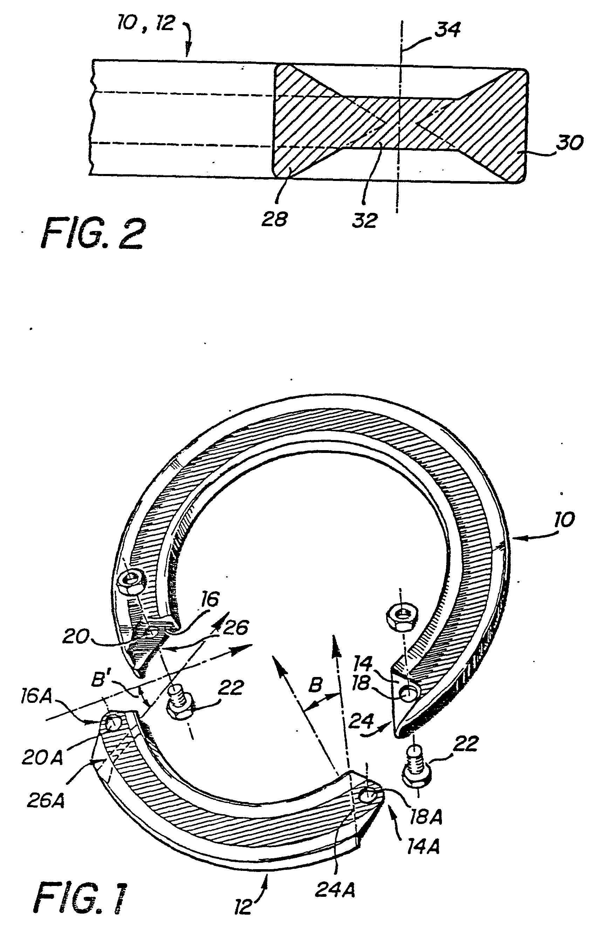

L'élément plus grand d'arceau 10 (fig. 1) fait approximativement les trois quarts (ou, alternativement, les deux tiers environ) d'un cercle, tandis que la pièce 12 a une longueur d'arc apte à compléter le cercle.

Les pièces 10 et 12 sont agencées à leurs extrémités circulaires pour pouvoir être fixées l'une à l'autre, formant ainsi le cercle complet. Dans ce but, les parties terminales 14, 16 de l'élément 10 sont découpées horizontalement au milieu du profil de l'élément, et les parties terminales correspondantes 14A et 16A, respectivement, de l'élément 12 le sont de la façon complémentaire. Les paires de trous 18, 18A et 20, 20A servent à la réunion des pièces 10 et 12 par des boulons 22. Cependant, les trous 18, 18A, 20 et 20A ont une fonction alternative qui sera décrite plus loin.

Les faces verticales 24, 24A et 26, 26A des extrémités partiellement coupées des deux éléments forment un angle B, B' avec le plan de la normale au même endroit. Les angles B, B' sont de préférence égaux. Les plans des faces 24A et 26A peuvent être parallèles ou former entre eux un angle ; le fait de prévoir les angles B, B' facilite grandement le montage et le démontage de l'élément 12 par rapport à l'élément 10. Si l'angle mentionné (non référencé) entre les dits plans des faces verticales est un angle aigu dont le sommet se trouve côté centre du cercle formé par les arceaux, la solidité du montage augmente. La solidité et la rigidité de la liaison des deux arceaux 10 et 12 sont également augmentées par le fait que les découpures 14, 14A et 16, 16A ne sont pas droites, mais coudées (voir fig. 1).

Le tore formé par le profil des éléments d'arceau est représenté en coupe verticale à la fig. 2. La section de l'arceau selon l'invention est constituée par deux sections partielles sous forme de triangles isocèles congruents 28 et 30, dont les sommets, confondus dans le profil de liaison et d'écartement (âme, entretoise) 32 rectangulaire, se regardent l'un l'autre. La section totale est donc symétrique par rapport au plan vertical cylindrique 34.

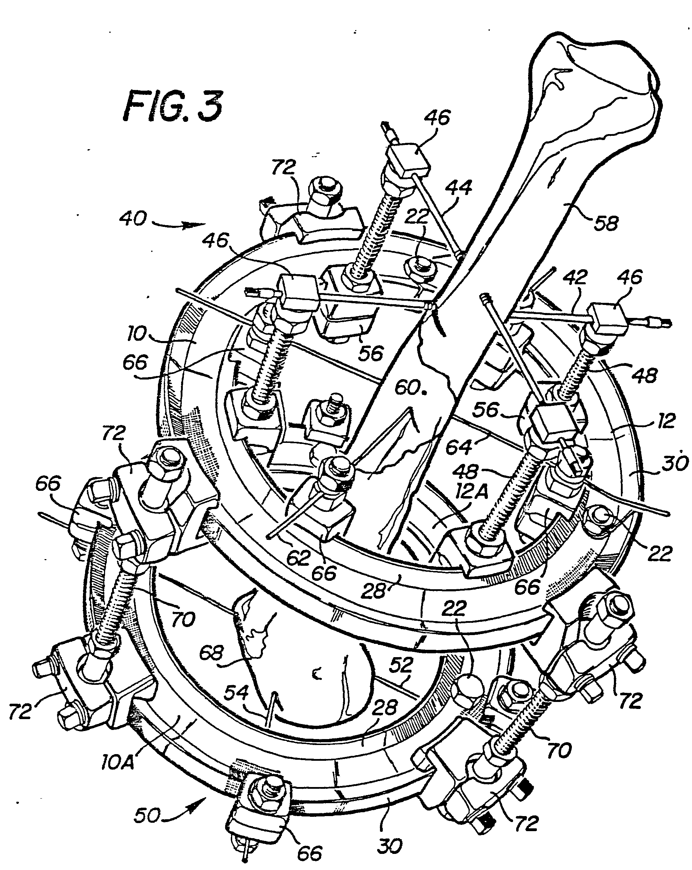

Le fixateur externe représenté à la fig. 3 sera maintenant décrit en détail. Pour les pièces, organes et éléments non décrits, ainsi que pour les généralités, référence est faite à la description du brevet no. 630 798.

Le fixateur externe pour fixation squelettique représenté dans la fig. 3 est mis en place comme suit :

Les deux groupes 40 et 50 de fiches 42, 44, respectivement 52, 54 sont mis en place à l'aide d'un gabarit ou guide et d'un vilebrequin non représentés. Les fiches du groupe 40 peuvent être introduites et serrées sans difficulté entre les mâchoires des pièces de fixation 46 des tiges ou fiches 42, 44. Les pièces de fixation 46 sont vissées au sommet des tiges filetées 48 montées aux pinces 56 ; ces pinces 56 sont serrées aux éléments d'arceau 10, 12, réunies par des boulons 22, à la section triangulaire intérieure 28 de l'arceau.

Les fiches transfixiantes 42, 44 tiennent en place le fragment supérieur 58 de l'os. Un fragment intermédiaire 60 est retenu par des fils transfixiants 62 et 64 tenus sous tension directement, sans tige, par des pinces 66 (dont la construction est décrite dans le brevet no. 630 798) serrées également sur la section intérieure 28 de l'arceau supérieur.

Un troisième fragment 68 de l'os est tenu en place par des fils transfixiants 52 et 54 directement supportés par les pinces 66 serrées sur la section profilée extérieure 30 de l'arceau inférieur 10A, 12A.

Les deux arceaux 10, 12 et 10A, 12A sont réunis à distance, de façon absolument rigide et solide, par trois ou quatre tiges filetées 70 qui sont fixées à la section triangulaire extérieure 30 des deux arceaux 10, 12 ; 10A, 12A par des pinces 72 ; ces pinces 72 sont agencées de façon à permettre un ajustement axial, donc une compression ou une extension de l'os.

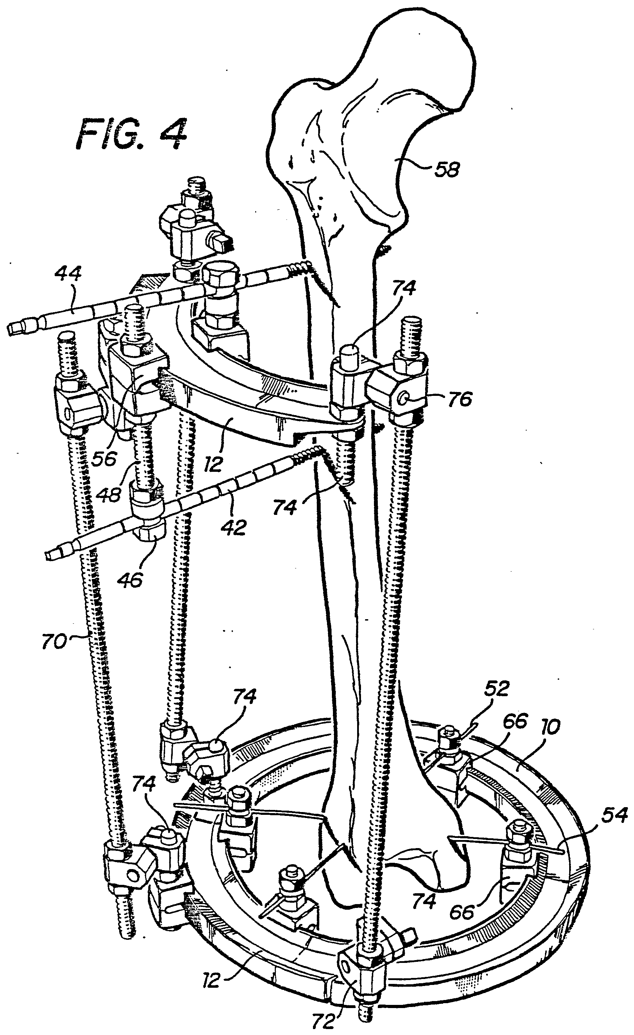

La fig. 4 montre l'utilisation d'un élément d'arceau partiel 12 sans qu'un cercle complet ne soit formé. Les trous 18A, 20A (fig. 1) ont servi à recevoir des boulons-tiges 74 servant au montage de pièces de liaison 76. Les autres éléments correspondent à ceux des fig. 1 à 3.

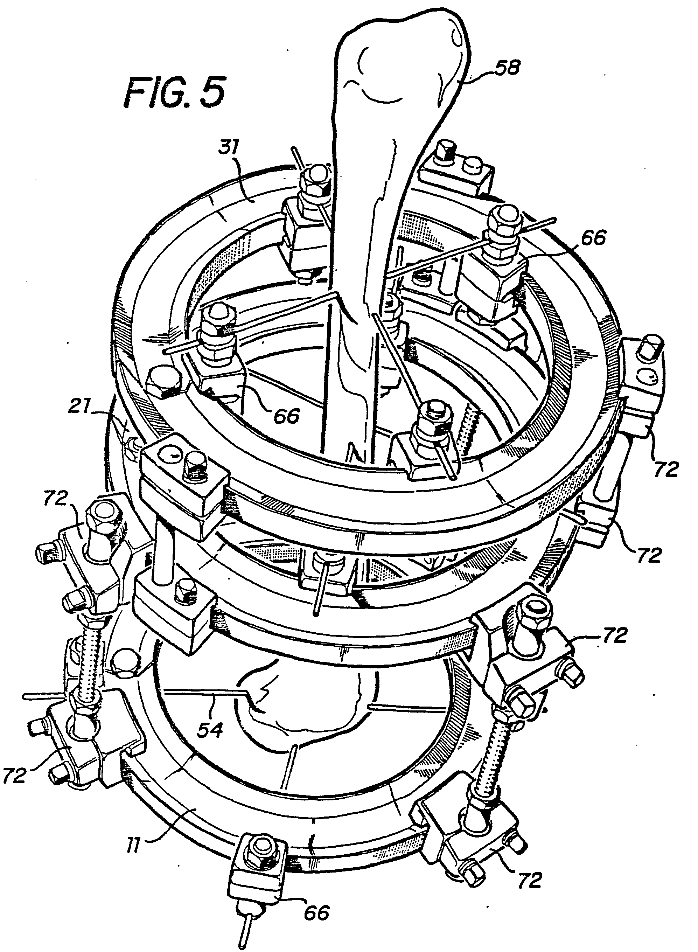

La fig. 5 représente un fixateur selon l'invention comportant trois éléments d'arceau à cercle complet 11, 21, 31. Ce fixateur sera utilisé pour l'ostéosynthèse de fractures compliquées multiples. Les autres éléments et pièces utilisés pour monter ce fixateur sont ceux déjà décrits. On voit ici spécialement le grand intérêt et les avantages particuliers que présentent l'élément d'arceau selon l'invention à section de serrage polygonale double, permettant la fixation d'organes aux pourtours extérieurs et intérieurs de l'arceau. Il est encore à noter que les différentes pinces (56, 66, 72) peuvent être utilisées grâce aux sections identiques et symétriques 28 et 30, indifféremment sur ces deux sections.

## Revendications

1. Fixateur externe pour ostéosynthèse et ostéoplastie comprenant au moins deux groupes d'au moins une fiche ou un fil maintenant chacun un fragment osseux, des tiges d'assemblages, au moins un élément d'arceau toroïdal ainsi que des organes de fixation des fiches ou fils et des tiges d'assemblage, caractérisé par le fait que la section dudit élément d'arceau comprend deux renforts latéraux (28, 30) de section polygonale réunis par un profil de liaison et d'écartement (32), lesdits renforts latéraux de section polygonale étant destinés à recevoir lesdits moyens de fixation, qui sont adaptés à être montés, déplacés librement, puis bloqués en toute position sur l'un et/ou l'autre des renforts constituant l'élément d'arceau.

2. Fixateur selon la revendication 1, caractérisé par le fait que ledit profil de liaison (32) a une forme générale rectangulaire.

3. Fixateur selon la revendication 1, caractérisé par le fait que lesdits renforts (28, 30) ont une section en forme de triangle isocèle dont les sommets sont dirigés l'un en regard de l'autre, dans le plan horizontal du tore.

4. Fixateur selon la revendication 1, caractérisé par le fait que ledit élément se présente sous forme d'une partie de cercle (10) ayant une longueur d'arc entre les deux tiers et les trois quarts d'un cercle.

5. Fixateur selon la revendication 1, caractérisé par le fait que ledit élément se présente sous forme d'une partie de cercle (12) ayant une longueur d'arc entre le quart et le tiers d'un cercle.

6. Fixateur selon les revendications 4 et 5, caractérisé par le fait que deux éléments (10, 12) ont des longueurs telles qu'ils forment ensemble un cercle complet, les extrémités des deux cercles partiels présentant des ouvertures (18, 20 ; 18A, 20A) pour le passage de pièces de liaison et étant coupées horizontalement et verticalement pour s'emboîter dans la partie correspondante de l'autre élément.

7. Fixateur selon la revendication 6, caractérisé par le fait que les extrémités (14, 16 ; 14A, 16A) des deux éléments sont complémentaires, pour permettre le montage du cercle par superposition des deux éléments.

8. Fixateur selon la revendication 7, caractérisé par le fait que chacune desdites découpures verticales est constituée par une découpure droite (24, 24A ; 26, 26A), sur l'une des parties horizontales, et par une découpure coudée sur l'autre.

9. Fixateur selon la revendication 8, caractérisé par le fait que ladite découpure droite (24, 24A ; 26, 26A) forme un angle par rapport à la normale au cercle, et que ladite découpure coudée consiste en une partie centrale arrondie entre deux prolongements sensiblement droits, de telle manière que lesdits prolongements droits viennent buter sur les prolongements droits de la partie correspondante en cas de contraintes radiales, vers l'intérieur ou l'extérieur.

## Claims

1. External fixation device for osteosynthesis and osteoplasty comprising at least two sets of at least one pin or one wire each retaining a bone portion, assembly rods, at least one arcuate element of toroid shape, and elements for fixing the pins or wires and the assembly rods, characterized in that the cross-section of the said arcuate element comprises two lateral reinforcements (28, 30) of polygonal cross-section united by way of a connecting and spacing profile (32), the said lateral reinforcements of polygonal cross-section being intended to receive the said fixing means, which are adapted to be mounted, displaced freely, and then locked in any position on the one and/or the other of the reinforcements constituting the arcuate element.

2. Fixation device according to Claim 1, characterized in that the said connecting profile (32) has a generally rectangular shape.

3. Fixation device according to Claim 1, characterized in that the said reinforcements (28, 30) have a cross-section in the shape of isosceles triangles whose apices are directed towards each other in the horizontal plane of the toroid.

4. Fixation device according to Claim 1, characterized in that the said element is in the form of a circle portion (10) having an arc length between twothirds and three-quarters of a circle.

5. Fixation device according to Claim 1, characterized in that the said element is in the form of a circle portion (12) having an arc length between a quarter and a third of a circle.

6. Fixation device according to Claims 4 and 5, characterized in that two elements (10, 12) have lengths such that together they form a complete circle, the ends of the two partial circles having openings (18, 20 ; 18A, 20A) for the passage of connecting pieces and being cut horizontally and vertically in order to engage in the corresponding part of the other element.

7. Fixation device according to Claim 6, characterized in that the ends (14, 16 ; 14A, 16A) of the two elements are complementary, in order to permit the assembly of the circle by superposition of the two elements.

8. Fixation device according to claim 7, characterized in that each of the said vertical cut is made up of a straight cut (24, 24A ; 26, 26A), on one of the horizontal parts, and by a bowed cut on the other.

9. Fixation device according to Claim 8, characterized in that the said straight cut (24, 24A ; 26, 26A) forms an angle relative to the perpendicular of the circle, and in that the said bowed cut consists of a rounded central part between two essentially straight extensions, in such a manner that the said straight extensions abut against the straight extensions of the corresponding part in the event of radial stresses, towards the inside or outside.

## Patentansprüche

1. Äußerer Spanner zur Osteosynthese und Osteoplastik, bestehend aus mindestens zwei Gruppen mindestens eines, jeweils ein Knochenfragment haltenden Stifts oder Drahtes, Verbindungsstangen, mindestens einem ringförmigen Bogensegment sowie Befestigungsmitteln für die Stifte oder Drähte und die Verbindungsstangen, dadurch gekennzeichnet, daß der Querschnitt besagten Bogensegments zwei seitliche Verdickungen (28, 30) polygonalen Querschnitts aufweist, die durch ein Verbindungs- und Abstandsprofil (32) verknüpft sind, wobei diese seitlichen Verdickungen polygonalen Querschnitts zur Aufnahme besagter Befestigungsmittel bestimmt sind, die zum Anbringen, zur freien Verschiebung und nachfolgenden Arretierung in beliebiger Stellung auf der einen und/ oder anderen der das Bogensegment darstellenden Verdickungen ausgebildet sind.

2. Spanner nach Anspruch 1, dadurch gekennzeichnet, daß besagtes Verbindungsprofil (32) eine allgemein rechteckige Gestalt besitzt.

3. Spanner nach Anspruch 1, dadurch gekennzeichnet, daß besagte Verdickungen (28, 30) im Querschnitt die Gestalt gleichschenkliger Dreiecke aufweisen, deren Scheitel in der horizontalen Ebene des Rings aufeinander weisen.

4. Spanner nach Anspruch 1, dadurch gekennzeichnet, daß besagtes Segment in der Gestalt eines Teilkreises (10) mit einer Bogenlänge zwischen zwei Dritteln und drei Vierteln eines Kreises vorliegt.

5. Spanner nach Anspruch 1, dadurch gekennzeichnet, daß besagtes Segment in der Gestalt eines Teilkreises (12) mit einer Bogenlänge zwischen einem Viertel und einem Drittel eines Kreises vorliegt.

6. Spanner nach Ansprüchen 4 und 5, dadurch gekennzeichnet, daß zwei Segmente (10, 12) so lang sind, daß sie zusammen einen vollständigen Kreis bilden, wobei die Enden der beiden Teilkreise Durchgangsöffnungen (18, 20 ; 18A, 20A) für Verbindungsstücke aufweisen und zum Einfügen in den entsprechenden Teil des anderen Segments horizontal und vertikal ausgeschnitten sind.

7. Spanner nach Anspruch 6, dadurch gekennzeichnet, daß die Enden (14, 16 ; 14A, 16A) der beiden Segmente komplementär sind, damit der Kreis durch Übereinanderlegen der beiden Segmente zusammengebaut werden kann.

8. Spanner nach Anspruch 7, dadurch gekennzeichnet, daß besagte vertikale Ausschnitte jeweils einen geraden Ausschnitt (24, 24A ; 26, 26A) auf einem der horizontalen Teile und einen gekrümmten Ausschnitt auf dem anderen darstellen.

9. Spanner nach Anspruch 8, dadurch gekennzeichnet, daß der besagte gerade Ausschnitt (24, 24A ; 26, 26A) mit der Kreisnormalen einen Winkel bildet und daß der besagte gekrümmte Ausschnitt aus einem mittleren, zwischen zwei im wesentlichen geraden Verlängerungen gerundeten Teil besteht, derart daß besagte gerade Verlängerungen sich im Fall radialer Spannungen nach innen oder außen auf den geraden Verlängerungen des entsprechenden Teils abstützen.

FIG. 2

FIG. 1

# FIG.3

FIG. 4

FIG.5